(19) Europäisches Patentamt
European Patent Office
Office européen des brevets

(11) Veröffentlichungsnummer: **0 079 512**
**B1**

(12) # EUROPÄISCHE PATENTSCHRIFT

(45) Veröffentlichungstag der Patentschrift: **23.04.86**

(21) Anmeldenummer: **82110066.6**

(22) Anmeldetag: **02.11.82**

(51) Int. Cl.⁴: **C 08 G 18/10**, C 08 G 18/50, C 08 G 18/83, C 07 C 125/06, C 07 C 127/19, C 07 C 127/24

(54) **Verfahren zur Herstellung von Polyaminen aus N-Monoaryl-N',N'-dialkylharnstoff-Verbindungen und ihre Verwendung zum Aufbau von Polyurethanen.**

(30) Priorität: **12.11.81 DE 3144874**

(43) Veröffentlichungstag der Anmeldung: **25.05.83 Patentblatt 83/21**

(45) Bekanntmachung des Hinweises auf die Patenterteilung: **23.04.86 Patentblatt 86/17**

(84) Benannte Vertragsstaaten: **BE DE FR GB IT NL**

(56) Entgegenhaltungen:
**EP - A - 0 050 275**
**EP - A - 0 061 627**
**DE - A - 2 546 536**
**DE - A - 2 948 419**

(73) Patentinhaber: **BAYER AG, Konzernverwaltung RP Patentabteilung, D-5090 Leverkusen 1 Bayerwerk (DE)**

(72) Erfinder: **Rasshofer, Werner, Dr., Wolfskaul 10, D-5000 Koeln 80 (DE)**
Erfinder: **Grögler, Gerhard, Dr., von-Diergardt-Strasse 46, D-5090 Leverkusen 1 (DE)**

**EP 0 079 512 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes Verfahren zur Herstellung von aromatischen Polyaminen, welche Urethan- und/oder Biuret- und/oder Harnstoff- und/oder Isocyanurat-Gruppen sowie vorzugsweise auch Ethergruppen enthalten, durch Hydrolyse von M-Monoaryl-N',N'-dialkylharnstoff-Verbindungen (1-Aryl-3,3-dialkylharnstoffe). Die Harnstoffe werden durch Reaktion von sekundären aliphatischen, cycloaliphatischen oder gesättigt heterocyclischen Aminen mit endständigen, an aromatische Reste gebundenen Isocyanatgruppen aufweisenden 2- bis 4-wertigen NCO-Verbindungen erhalten und weisen die allgemeine Formel

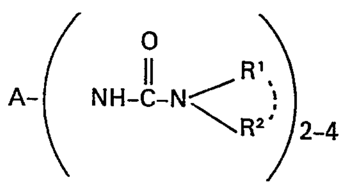

auf, wobei

$R^1$, $R^2$ (Cyclo)alkylreste oder zusammen einen Ring bildenden Rest und

A einen 2- bis 4-wertigen Rest, der mit aromatischen Gruppen an die Harnstoffe gebunden ist, darstellen. Die Hydrolyse erfolgt in Gegenwart von Wasser und bis 2 Äquivalenten einer Hydroxid-Ionen besitzenden Base und/oder bis zu 2 Äquivalenten einer tert.-Aminogruppen aufweisenden Verbindung, gegebenenfalls in Gegenwart von Lösungsmitteln. Weiterhin wird die Verwendung der so gewonnenen Polyamine zum Aufbau von Polyurethanen beansprucht.

Es ist bekannt, dass aromatische Isocyanate durch saure Hydrolyse in primäre Amine überführt werden können. Die Reaktion verläuft allerdings nur sehr unvollständig, da das bei der Hydrolyse gebildete Amin mit noch nicht umgesetztem Isocyanat zum entsprechenden Harnstoff weiter reagiert. Diese Folgereaktion lässt sich auch durch Anwendung überschüssiger starker Mineralsäure nicht unterdrücken. Ein neueres Beispiel findet sich in der JP-PS 55 007 827.

Es ist auch bekannt, dass aromatische Isocyanate durch basische Hydrolyse in aromatische Amine überführt werden können. Die bei H. John, J. Prakt. Chemie 130, 314 f. (1931) und H. John, J. Prakt. Chemie, 130, 323 f. (1931) für zwei bestimmte heterocyclische Isocyansäureester genannten Hydrolysebedingungen sind aber für die Überführung von NCO-Prepolymeren in die entsprechenden Polyamine völlig ungeeignet.

In den Patentanmeldungen DE-OS 2 948 419 und P. 3 039 600.0 werden Verfahren beschrieben, aus den durch Reaktion von NCO-Verbindungen mit wässrigen Basen zugänglichen Carbamatsalzen durch acidolytische Zersetzung mit Mineralsäuren oder starken organischen Säuren die freien Aminoverbindungen herzustellen. Nachteilig an diesen Verfahren ist, dass zwangsläufig z.B. mineralsaure Salze anfallen, deren Beseitigung die Wirtschaftlichkeit der genannten Verfahren beeinträchtigt.

In der DE-B 1 270 046 wird ein Verfahren zur Herstellung definierter, Polyalkylenglykolether-Segmente enthaltender, primärer aromatischer Amine beschrieben, bei dem man Umsetzungsprodukte von aromatischen Di- oder Triisocyanaten mit Polyalkylenglykolethern und/oder Polyalkylenglykolthioethern, vorzugsweise solchen mit Molekulargewichten zwischen 400 und 4000, mit sekundären oder tertiären Carbinolen umsetzt und anschliessend (gegebenenfalls in Gegenwart saurer Katalysatoren) in einem inerten Lösungsmittel einer thermischen Spaltung unterwirft. Nachteilig ist dabei, dass im Verlauf der thermischen Spaltung der Urethane brennbare, leicht flüchtige Alkene entstehen, die im Gemisch mit Luft explosiv sind, so dass entsprechende Vorsichtsmassnahmen getroffen werden müssen.

Gegenstand der DE-B 1 694 152 ist die Herstellung von mindestens zwei endständige Aminogruppen aufweisenden Prepolymeren durch Umsetzung von Hydrazin, Aminophenylethylamin oder anderen Diaminen mit einem NCO-Prepolymer aus einem Polyetherpolyol und Polyisocyanat (NCO:NH-Verhältnis = 1:1,5 bis 1:5). Nichtumgesetztes Amin muss dabei in einem weiteren Schritt sorgfältig entfernt werden, da es die Umsetzung mit Polyisocyanaten stark katalysiert, so zu kurzen Verarbeitungszeiten führt und auch selbst als Reaktionspartner auftritt.

Eine andere Synthesemöglichkeit für Urethangruppen aufweisende Polyamine wird in der FR-PS 1 415 317 beschrieben. Urethangruppenhaltige NCO-Prepolymere werden mit Ameisensäure in die N-Formylderivate überführt, die zu endständigen aromatischen Aminen verseift werden. Auch die Reaktion von NCO-Prepolymeren mit Sulfaminsäure gemäss DE-B 1 555 907 führt zu Verbindungen mit endständigen Aminogruppen. Des weiteren werden höhermolekulare, aliphatische, sekundäre und primäre Aminogruppen aufweisende Voraddukte nach DE-B 1 215 373 durch Umsetzung höhermolekularer Hydroxylverbindungen mit Ammoniak in Gegenwart von Katalysatoren unter Druck bei hohen Temperaturen erhalten oder gemäss US-PS 3 044 989 durch Umsetzung höhermolekularer Polyhydroxylverbindungen mit Acrylnitril unter anschliessender katalytischer Hydrierung. Auch durch Umsetzung von NCO-Prepolymeren mit Hydroxygruppen aufweisenden Enaminen, Aldiminen oder Ketiminen und anschliessende Hydrolyse erhält man gemäss DE-A 2 546 536 bzw. US-PS 3 865 791 höhermolekulare, endständige Aminogruppen und Urethangruppen aufweisende Verbindungen. Nachteilig ist vor allem, dass durch Hydrolyse freigesetztes z.B. aromatisches Amin mit z.B. dem Ketimin-NCO-Addukt unter Harnstoffbildung reagiert und dass die Hydrolyse in den meisten Fällen nur sehr langsam erfolgt.

Eine weitere Synthesemöglichkeit für Urethan- und Ethergruppen aufweisende aromatische Polyamine liegt in der bei der Reaktion von Isatosäureanhydrid und Diolen eintretenden Ringöff-

nung. Solche Polyamine sind z.B. beschrieben in der US-PS 4 180 644, den DE-A 2 619 840, 2 648 774, 2 648 825 und 2 019 432. Nachteilig für viele Zwecke ist die geringe Reaktivität der solcherart erhaltenen, aromatischen Esteramine. Auch die Reaktion von Nitroarylisocyanaten mit Polyolen mit nachfolgender Reduktion zu den Aminen ist bekannt (US-PS 2 888 439). Nachteilig sind hier vor allem die hohen Kosten des Reduktionsschritts.

Überraschenderweise wurde gefunden, dass man definierte, Urethan- und/oder Harnstoff- und/oder Biureto- und/oder Isocyanurat- und bevorzugt auch Ethergruppen (oder andere Gruppen, z.B. Acetal-, Thioether-, Dimethylsiloxan- oder Polybutadien-Gruppen) enthaltende aromatische Polyamine erhält, wenn man nachstehend näher beschriebene Harnstoffe mit einer Base, vorzugsweise in äquivalenten oder leicht überschüssigen Mengen (bezogen auf die Menge an Harnstoffgruppen), behandelt.

Es ist überraschend, dass die basische Hydrolyse der erfindungsgemäss substituierten Harnstoffgruppen so selektiv an mit (cyclo)-aliphatischen Resten substituierten Harnstoffpartialstrukturen erfolgt, dass gleichfalls anwesende Urethan- und/oder Biuret- und/oder Allophanat- und/oder Isocyanuratgruppen nicht gespalten werden. Dies war dem veröffentlichten Stand der Technik nicht zu entnehmen. Darüber hinaus ist es überraschend, dass die an sich bekannte Hydrolyse monomerer, niedermolekularer Monoharnstoffe sich sogar glatt auf höher molekulare Polyharnstoff-Verbindungen übertragen lässt. Vorteilhaft ist, dass gegenüber bekannten Hydrolyseverfahren von NCO-Verbindungen durch die Vorreaktion der NCO-Verbindung mit sek. Aminen, die quantitativ zu Harnstoffverbindungen führt, keine Vorverlängerung über NCO-Wasser-Reaktion eintritt. Ausserdem sind die N′,N′-Dialkylharnstoff-Derivate gewissermassen lagerstabile Derivate der in vielen Fällen nicht lagerbeständigen NCO-Voraddukte und können daher zu einem beliebigen Zeitpunkt nach ihrer Herstellung in die gewünschten Polyamine überführt werden.

Gegenstand der Erfindung ist somit ein Verfahren zur Herstellung von Urethan- und/oder Harnstoff- und/oder Biuret- und/oder Allophanat- und/oder Isocyanurat- sowie bevorzugt auch Ethergruppen enthaltenden aromatischen Polyaminen, dadurch gekennzeichnet, dass eine Verbindung der allgemeinen Formel

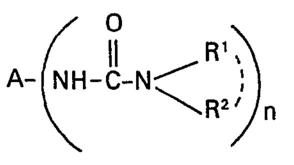

wobei

A ein n-wertiger Rest ist, wie er durch Entfernung von n Isocyanatgruppen aus einer Urethan- und/oder Harnstoff- und/oder Biuret- und/oder Allophanat- und/oder Isocyanurat- sowie besonders auch Ethergruppen enthaltenden NCO-Verbindung mit aromatisch gebundenen NCO-Gruppen, vorzugsweise einem NCO-Prepolymer auf Basis aromatischer Polyisocyanate, entsteht,

$R^1$ und $R^2$ unabhängig voneinander einen 1 bis 10 Kohlenstoffatome enthaltenden linearen oder verzweigten oder cyclischen gesättigten Kohlenwasserstoffrest bedeuten oder zusammen einen Ring mit 4 bis 6 Ring-C-Atomen bilden und

n eine ganze Zahl von 2 bis 4 bedeutet, unter Kohlendioxyd-Abspaltung einer basischen Hydrolyse unterworfen wird.

Gegenstand der vorliegenden Erfindung ist ferner ein Verfahren zur Herstellung von Urethan- und/oder Harnstoff- und/oder Biuret- und/oder Allophanat- und/oder Isocyanurat- sowie (bevorzugt auch) Ethergruppen enthaltenden aromatischen Polyaminen, dadurch gekennzeichnet, dass eine Verbindung der allgemeinen Formel

$$A\text{-}(NCO)_n,$$

wobei

A ein Rest ist, wie er durch Entfernung von n Isocyanatgruppen aus einer Urethan- und/oder Harnstoff- und/oder Biuret- und/oder Allophanat- und/oder Isocyanurat- sowie bevorzugt Ethergruppen enthaltenden NCO-Verbindung mit an aromatische Reste gebundenen NCO-Gruppen entsteht, und

n eine ganze Zahl von 2 bis 4 ist, gegebenenfalls gelöst in einem NCO-inerten Lösungsmittel, in der Wärme mit einem Gemisch aus

a) Wasser,

b) 0,1 bis 10 Äquivalenten (bezogen auf 1 Äquivalent NCO) eines sekundären Amins der allgemeinen Formel

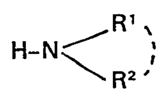

wobei

$R^1$ und $R^1$ unabhängig voneinander jeweils einen 1 bis 10 Kohlenstoffatome enthaltenden linearen oder verzweigten aliphatischen oder cycloaliphatischen, gesättigten Kohlenwasserstoffrest bedeuten oder zusammen für einen 4 bis 6 Ring-C-Atome enthaltenden heterocyclischen Ring stehen,

c) 0 bis 2 Äquivalente einer Hydroxidionen besitzenden Base (bezogen auf 1 Äquivalent NCO),

d) 0 bis 2 Äquivalenten einer tert.-Aminogruppen aufweisenden Verbindung (bezogen auf 1 Äquivalent NCO) und

e) 0 bis 100 Teilen eines Lösungsmittels (bezogen auf 1 Teil der Verbindung $A(NCO)_n$),

worin A und n wie oben definiert sind, wobei von den Komponenten c) und d) mindestens eine in einer Menge von 0,1 bis 2 Äquivalenten, bevorzugt 0,8 bis 1,8 Äquivalenten, besonders bevorzugt 1,0 bis 1,5 Äquivalenten, zum Einsatz kommt, behandelt und das entstandene aromatische Polyamin auf an sich bekannte Weise aus dem Reaktionsprodukt isoliert wird.

Gegenstand der Erfindung ist ferner die Verwendung der nach dem erfindungsgemässen Verfahren zugänglichen aromatischen Polyamine zur Herstellung von gegebenenfalls zellförmigen Polyurethankunststoffen und Polyurethanschaumstoffen durch Umsetzung von

I Polyisocyanaten mit
II Polyaminen, sowie gegebenenfalls
III weiteren niedermolekularen und/oder höhermolekularen Verbindungen mit gegenüber Isocyanaten reaktiven Gruppen, gegebenenfalls
IV in Anwesenheit an sich bekannter Hilfs- und Zusatzstoffe,

dadurch gekennzeichnet, dass als Komponente II die nach dem erfindungsgemässen Verfahren hergestellten Polyamine eingesetzt werden.

Die im erfindungsgemässen Verfahren einsetzbaren NCO-Verbindungen $A(NCO)_n$ werden in an sich bekannter Weise durch Umsetzung von Wasser und/oder hoch- und/oder niedermolekularen, Hydroxy- und/oder Amino- und/oder Thiolgruppen enthaltenden Verbindungen (Molekulargewicht: 60–12 000) mit einem Überschuss an Polyisocyanat hergestellt.

Es kommen hierfür als Polyisocyanate im Prinzip alle aromatischen und heteroaromatischen Polyisocyanate in Frage, beispielsweise 1,3- und 1,4-Phenylendiisocyanat, 2,4- und 2,6-Toluylendiisocyanat, sowie beliebige Gemische dieser Isomeren, Diphenylmethan-2,4'- und/oder -4,4'-diisocyanat, einschliesslich seiner Alkyl- und Chlor-substituierten Derivate und Naphthylen-1,5-diisocyanat.

Ferner kommen beispielsweise als aromatische Polyisocyanate in Frage: Triphenylmethan-4,4',4"-triisocyanat, Polyphenyl-polymethylen-polyisocyanate, wie sie durch Anilin-Formaldehyd-Kondensation und anschliessende Phosgenierung erhalten und z.B. in den GB-PSen 874 430 und 848 671 beschrieben werden, m- und p-Isocyanatophenylsulfonyl-isocyanate gemäss der US-PS 3 454 606, perchlorierte Arylpolyisocyanate, wie sie z.B. in der DE-Auslegeschrift 1 157 601 (US-PS 3 277 138) beschrieben werden, Isocyanuratgruppen aufweisende Polyisocyanate, wie sie z.B. in der US-PS 3 001 973, in den DE-PSen 1 022 789, 1 222 067 und 1 027 394 sowie in den DE-PSen 1 929 034 und 2 004 048 beschrieben werden, Urethangruppen aufweisende Polyisocyanate, wie sie z.B. in der BE-PS 752 261 oder in den US-PSen 3 394 164 und 3 644 457 beschrieben werden, acylierte Harnstoffgruppen aufweisende Polyisocyanate gemäss der DE-PS 1 230 778 sowie durch Telomerisationsreaktionen hergestellte Polyisocyanate, wie sie z.B. in der US-PS 3 654 196 beschrieben werden, ferner auch Uretdion-polyisocyanate oder Isocyanurat-polyisocyanate.

Geeignete Ausgangspolyisocyanate sind auch die nach DE-A 2 922 966 zugänglichen, Schwefel enthaltenden aromatischen Polyisocyanate.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Besonders bevorzugt werden in der Regel die technisch leicht zugänglichen Polyisocyanate, z.B. das 2,4- und 2,6-Toluylendiisocyanat sowie beliebige Gemische dieser Isomeren («TDI»), Polyphenyl-polymethylen-polyisocyanate, wie sie durch Anilin-Formaldehyd-Kondensation und anschliessende Phosgenierung hergestellt werden. («rohes MDI»), sowie insbesondere Urethangruppen, Isocyanuratgruppen oder Harnstoffgruppen aufweisende Polyisocyanate («modifizierte Polyisocyanate»), insbesondere solche modifizierten Polyisocyanate, die sich vom 2,4- und/oder 2,6-Toluylendiisocyanat bzw., vom 4,4'- und/oder 2,4'-Diphenylmethandiisocyanat ableiten.

Für die Herstellung des NCO-Prepolymeren werden vorzugsweise Verbindungen mit einem Molekulargewicht von 400 bis 12 000, insbesondere 400 bis 6000, eingesetzt, welche mindestens 2, vorzugsweise 2 bis 4, insbesondere 2 oder 3, Hydroxyl-, Amino- und/oder Thiolgruppen (bevorzugt Hydroxylgruppen) aufweisen und frei an leicht hydrolysierbaren Gruppen wie z.B. Estergruppen sind. In Frage kommen beispielsweise die in der Polyurethanchemie üblichen Polyacetale, Polythioether und insbesondere Polyether.

Die erfindungsgemäss in Frage kommenden, mindestens 2, in der Regel 2 bis 8, vorzugsweise 2 bis 4, Hydroxylgruppen aufweisenden Polyether sind solche der an sich bekannten Art und werden z.B durch Polymerisation von Epoxiden wie Ethylenoxid, Propylenoxid, Butylenoxid, Tetrahydrofuran, Styroloxid oder Epichlorhydrin mit sich selbst, z.B. in Gegenwart von Lewis-Katalysatoren wie $BF_3$, oder durch Anlagerung dieser Epoxide, vorzugsweise von Ethylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z.B. Ethylenglykol, Propylenglykol-(1,3) oder -(1,2), Trimethylolpropan, Glycerin, Sorbit, 4,4'-Dihydroxy-diphenylpropan, Anilin, Ethanolamin oder Ethylendiamin hergestellt. Auch Sucrosepolyether, wie sie z.B. in den DE-B 1 176 358 und 1 064 938 beschrieben werden, sowie auf Formit oder Formose gestartete Polyether (DE-A 2 639 083 bzw. 2 737 951), kommen erfindungsgemäss in Frage. Vielfach sind solche Polyether bevorzugt, die überwiegend (bis zu 90 Gew.-% bezogen auf alle vorhandenen OH-Gruppen im Polyether) primäre OH-Gruppen aufweisen.

Unter den Polythioethern seien insbesondere die Kondensationsprodukte von Thiodiglykol mit sich selbst und/oder mit anderen Glykolen angeführt.

Auch bereits Urethan- oder Harnstoffgruppen enthaltende Polyhydroxylverbindungen sowie gegebenenfalls modifizierte natürliche Polyole sind verwendbar. Auch Anlagerungsprodukte

von Alkylenoxiden an Phenol-Formaldehyd-Harze oder auch an Harnstoff-Formaldehyd-Harze sind erfindungsgemäss einsetzbar.

Die genannten Polyhydroxylverbindungen können vor ihrer Verwendung noch in der verschiedensten Weise modifiziert werden: So lässt sich gemäss DE-A 2 210 839 (US-PS 3 849 515) und 2 544 195 ein Gemisch aus verschiedenen Polyhydroxylverbindungen durch Veretherung in Gegenwart einer starken Säure zu einem höhermolekularen Polyol kondensieren, welches aus über Etherbrücken verbundenen verschiedenen Segmenten aufgebaut ist. Es ist auch möglich, z.B. gemäss DE-A 2 559 372 in die Polyhydroxylverbindungen Amidgruppen einzuführen.

Vertreter der genannten erfindungsgemäss zu verwendenden Verbindungen sind z.B. in High Polymers, Vol. XVI, «Polyurethanes, Chemistry and Technology», verfasst von Saunders-Frisch, Interscience Publishers, New York, London, Band I, 1962, Seiten 32 bis 42 und Seiten 44 bis 54 und Band II, 1964, Seiten 5 und 6 und 198 und 199, sowie im Kunststoffhandbuch, Band VII, Vieweg-Höchtlen, Carl-Hanser-Verlag, München, 1966, z.B. auf den Seiten 45 bis 71, beschrieben. Selbstverständlich können Mischungen der obengenannten Verbindungen mit mindestens 2 gegenüber Isocyanaten reaktionsfähigen Wasserstoffatomen mit einem Molekulargewicht von 400 bis 12 000, z.B. Mischungen von verschiedenen Polyethern, eingesetzt werden.

Für die Herstellung des im erfindungsgemässen Verfahren einzusetzenden NCO-Prepolymeren kommen, gebenenfalls nur anteilsweise, als Ausgangskomponenten auch relativ niedermolekulare Verbindungen mit mindestens 2 gegenüber Isocyanaten reaktionsfähigen Wasserstoffatomen und einem Molekulargewicht von 18 bis 399, vorzugsweise 60 bis 399, in Betracht. Auch in diesem Fall versteht man hierunter Wasser und/oder Hydroxylgruppen und/oder Aminogruppen und/oder Thiolgruppen, vorzugsweise Hydroxylgruppen aufweisende Verbindungen, wie sie als Kettenverlängerungsmittel oder Vernetzungsmittel aus der Polyurethanchemie an sich bekannt sind. Diese Verbindungen weisen in der Regel 2 bis 8, vorzugsweise 2 bis 4, gegenüber Isocyanaten reaktionsfähige Wasserstoffatome auf.

Auch in diesem Fall können Mischungen von verschiedenen Verbindungen mit mindestens 2 gegenüber Isocyanaten reaktionsfähigen Wasserstoffatomen mit einem Molekulargewicht von 18 bis 399 verwendet werden.

Als Beispiele für derartige Verbindungen seien genannt: Wasser, Ethylenglykol, Propandiol-(1,2) und -(1,3), Butandiol-(1,4) und -(2,3), Pentandiol-(1,5), Hexandiol-(1,6), Octandiol-(1,8), Neopentylglykol, 1,4-Bishydroxymethyl-cyclohexan, 2-Methyl-1,3-propandiol, Dibrombutendiol (US-PS 3 723 392), Dianhydromannit und Dianhydrosorbit, Glycerin, Trimethylolpropan, Hexantriol-(1,2,6), Trimethylolethan, Pentaerythrit, Chinit, Mannit und Sorbit, Ricinusöl, Di-, Tri- und Tetraethylenglykol, Di-, Tri- und Tetra-propylenglykol, Dibutylenglykol und höhere Polyethylen-, Polypropylen- oder Polybutylenglykole mit einem Molekulargewicht bis 399, 4,4'-Dihydroxy-diphenylpropan, Di-hydroxymethyl-hydrochinon, Ethanolamin, Diethanolamin, N-Methyldiethanolamin, Triethanolamin und 3-Aminopropanol.

Als niedermolekulare Polyole kommen auch die Gemische von Hydroxyaldehyden und Hydroxyketonen («Formose») bzw. die hieraus durch Reduktion erhaltenen mehrwertigen Alkohole («Formit») in Frage, wie sie bei der Selbstkondensation von Formaldehydhydrat in Gegenwart von Metallverbindungen als Katalysator und von zur Endiolbildung befähigten Verbindungen als Co-Katalysator entstehen (DE-A 2 639 084, 2 714 084, 2 714 104, 2 271 186, 2 738 154 und 2 738 512).

Erfindungsgemäss geeignete aliphatische Diamine sind beispielsweise Ethylendiamin, 1,4-Tetramethylendiamin, 1,6-Hexamethylendiamin, 1,12-Dodecamethylendiamin sowie deren Gemische, 1-Amino-3,3,5-trimethyl-5-aminomethylcyclohexan («Isophorondiamin»), 2,4- und 2,6-Hexahydrotoluylendiamin sowie deren Gemische, Perhydro-2,4'- und -4,4'-diaminodiphenylmethan, p-Xylylendiamin, Bis-(3-aminopropyl)-methylamin, Diaminoperhydroanthrazene (DE-A 2 638 731) und cycloaliphatische Triamine gemäss DE-A 2 614 244. Auch Hydrazin und substituierte Hydrazine, z.B. Methylhydrazin, kommen erfindungsgemäss in Betracht.

Als Beispiele für aromatische Diamine seien die Ethergruppen aufweisenden Diamine gemäss DE-A 1 770 525 und 1 809 172 (US-PSen 3 654 364 und 3 736 295), gegebenenfalls in 5-Stellung substituierte 2-Halogen-1,3-phenylendiamine (DE-A 2 001 772, 2 025 896 und 2 065 869), 3,3'-Dichlor-4,4'-diamino-diphenylmethan, Toluylendiamin, 4,4'-Diaminodiphenylmethan, 4,4'-Diaminodiphenyldisulfide (DE-A 2 404 976), Diaminodiphenyldithioether (DE-A 2 509 404), durch Alkylthiogruppen substituierte aromatische Diamine (DE-A 2 638 760), Sulfonat- oder Carboxylatgruppen enthaltende aromatische Diamine (DE-A 2 720 166) sowie die in der DE-A 2 635 400 aufgeführten hochschmelzenden Diamine genannt. Beispiele für aliphatisch-aromatische Diamine sind die Aminoalkylthioaniline gemäss DE-A 2 734 574.

Die freie Isocyanatgruppen aufweisenden Prepolymeren werden in an sich bekannter Weise durch Umsetzung der Reaktionspartner in der Schmelze oder in Lösung hergestellt. Das Äquivalentverhältnis von NCO-Gruppen zu aktiven Wasserstoffatomen (bevorzugt OH-Gruppen) ist in jedem Fall grösser als 1, soll in der Regel zwischen 1,5:1 und 2,8:1 liegen. Selbstverständlich ist es möglich, einen noch grösseren Überschuss an Polyisocyanat zu verwenden. Die Voraddukte haben je nach den gewählten Ausgangskomponenten im allgemeinen ölige bis wachsartige Konsistenz. Beträgt das NCO/OH-Verhältnis mehr als 2, so erhält man im wesentlichen nicht verlängerte Voraddukte, während NCO/OH-Verhältnisse von unter 2 eine Erhöhung des mittleren Molekulargewichts der Voraddukte zur Folge haben. Es ist, wie schon erläutert, auch möglich,

niedermolekulare Polyole als Kettenverlängerungsmittel bei der Herstellung der Prepolymere anteilmässig neben höhermolekularen Ausgangsverbindungen mitzuverwenden; in diesem Fall erhält man ebenfalls höhermolekulare Voraddukte. Enthalten die NCO-Prepolymeren weitere Gruppierungen, z.B. Carbodiimid-, Acylharnstoff- oder Uretonimingruppen, so können diese gegebenenfalls auch in den erfindungsgemässen Produkten enthalten sein.

Für das erfindungsgemässe Verfahren werden Prepolymere bevorzugt, die aus höhermolekularen Polyetherglykolen, gegebenenfalls unter Mitverwendung von Kettenverlängerern der oben beschriebenen Art, und aliphatischen und/oder aromatischen Diisocyanaten im Äquivalentverhältnis von 1:1,5 bis 1:2,0, insbesondere ca. 1:2, erhalten worden sind.

Für das erfindungsgemässe Verfahren geeignete sekundäre Amine b) der allgemeinen Formel

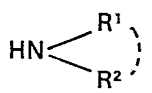

sind z.B. Dimethylamin, Diethylamin, Di-i-propylamin, Diisobutylamin, Di-n-propylamin, Di-n-butylamin, Di-sec.-butylamin, Di-iso-amylamin, Di-n-octylamin, Di-2-ethylhexylamin, Di-n-nonylamin. Weniger geeignet sind Verbindungen mit zwei oder mehr sekundären Aminogruppen wie z.B. N,N′-Dimethylethylendiamin, N,N′-Dimethylhexan-1,6-diamin, N,N′-Dimethylbutan-1,4-diamin, N,N′,N″-Trimethyldiethylentriamin.

Ebenfalls geeignet, aber aus Gründen der Zugänglichkeit weniger bevorzugt sind sekundäre Amine mit unterschiedlicher Substitution wie z.B. Methylpropylamin oder Verbindungen, die im gleichen Molekül eine sekundäre und eine tertiäre Aminogruppe wie z.B. Trimethylethylendiamin aufweisen.

Als Hydroxidionen besitzende Basen c) sind organische und anorganische Hydroxidionen enthaltende oder -bildende Verbindungen geeignet. Als organische Basen seien die Tetraalkylammoniumhydroxide und auf einem unlöslichen Polymerskelett fixierte Trialkylammoniumhydroxid-Reste angeführt, bevorzugt sind jedoch die anorganischen Alkali- und Erdalkalimetallhydroxide und Erdalkalioxide, sowie Alkalicarbonate und Alkalihydrogencarbonate. Besonders bevorzugt sind Natrium- und Kaliumhydroxid.

Als tert.-Aminogruppen aufweisende Verbindungen d) sind eine ganze Reihe verschiedener Verbindungen geeignet, beispielsweise einfache Trialkylamine mit gleicher oder unterschiedlicher Substitution wie Tri-ethylamin, Tri-i-propylamin, Tri-iso-butylamin, Cyclohexyl-di-i-propylamin.

Ebenfalls geeignet sind peralkylierte Polyamine wie z.B. Tetramethylethylendiamin, Tetramethylbutylentriamin, Tetramethylhexylendiamin, Permethyldiethylentriamin, Permethyldipropylentriamin, Permethyltriethylentetramin, 1,4-Dimethylpiperazin, 1-Methyl-4-(2-dimethylaminoethyl)-piperazin, N-(2-dimethylaminoethyl)-morpholin, Bis-(2-diethylaminoethyl)-ether, Bis-(2-dimethylaminopropyl)-ether und Diazabicyclooctan.

Ebenfalls geeignet, aber weniger bevorzugt, sind aromatische und heteroaromatische Amine wie z.B. N,N-Dimethylanilin, N,N-Dimethyl-o-toluidin oder Pyridin, Picolin, Lutidine oder auch Amidine wie Diazabicycloundecen.

Durchführung des erfindungsgemässen Verfahrens

Variante A (allgemeines Verfahren)

Durch Zugabe eines sekundären Amins, wie sie oben beispielhaft aufgeführt worden sind, zu einer vorgelegten NCO-Verbindung wird der entsprechende Harnstoff hergestellt. Die NCO-Verbindung kann dabei als Lösung in oder als Gemisch mit einem NCO-inerten Lösungsmittel vorliegen. Geeignete Lösungsmittel sind z.B. Dichlormethan, Trichlormethan, Tetrachlormethan, 1.1.1-Trichlorethan, Cyclohexan, Methylcyclohexan, Hexan, Diethylether, Benzol, Toluol etc., bevorzugt aber wassermischbare Solventien wie Dimethoxyethan, Tetrahydrofuran, Dioxan. Insgesamt ist aber die Verwendung von Lösungsmitteln für das NCO-Prepolymer weniger bevorzugt. Das sekundäre Amin, das bevorzugt etwa bei Raumtemperatur zugegeben wird, wird entweder in reiner Form oder als Mischung und/oder als Lösung in einem geeigneten Solvens zugegeben. Als solches ist Wasser bevorzugt. Das sekundäre Amin wird in einer den vorhandenen NCO-Gruppen mindestens equimolaren Menge eingesetzt, so dass das Äquivalentverhältnis NH:NCO$\geq$1 ist. Es ist problemlos möglich, einen Überschuss an Amin (z.B. NH:NCO = 2:1) zu verwenden, so dass das überschüssige sekundäre Amin gleichzeitig als Verdünner fungiert.

Die zu Harnstoffen führende Reaktion verläuft unter Wärmeabgabe, durch externe Kühlung wird sichergestellt, dass die Temperatur ca. 60 bis 70°C nicht überschreitet. Die bevorzugten und im Rahmen des erfindungsgemässen Verfahrens bevorzugt eingesetzten Harnstoffverbindungen stellen harzartige bis flüssige, in organischen Lösungsmitteln lösliche Verbindungen dar.

Nachdem die Harnstoff-Reaktionsmischung keinen messbaren NCO-Wert mehr aufweist, wird Wasser zugesetzt, sofern die Zugabe des Amins zur vorgelegten NCO-Verbindung nicht in wässriger Mischung/Lösung vorgenommen wurde. Die Menge des Wassers richtet sich nach der Menge der vorhandenen Harnstoffgruppen. Pro Mol Harnstoffgruppe wird mindestens 1 Mol Wasser eingesetzt, ein z.B. 1,1- bis 100-facher Überschuss ist bevorzugt.

Zu dieser Reaktionsmischung, die neben der Harnstoffverbindung und Wasser noch weitere Lösungsmittel bzw. Verdünner sowie Emulgatoren zur Verbesserung der Amin/NCO-Verbindungs-Reaktion enthalten kann, wird nun Base zugegeben.

Katalytische Mengen $OH^{\ominus}$-Ionen haben keinen Effekt, da sie zu unwirksamen $HCO_3^{\ominus}$-Anionen

umgewandelt werden. Tertiäre Amine können zwar in katalytischen bzw. unterstöchiometrischen Mengen (bezogen auf die Menge der Harnstoffgruppen) erfolgreich eingesetzt werden, doch ist die Reaktionsgeschwindigkeit relativ gering, so dass eine ungünstige Raum/Zeit-Ausbeute resultiert. Da ohnehin zur Aufarbeitung destilliert werden muss, bringt die Verwendung unterstöchiometrischer Mengen tert.-Aminogruppen aufweisender Verbindungen keine besonderen Vorteile.

Es werden also im Falle von Hydroxid-Ionen aufweisenden Verbindungen zwingend und im Falle von tert.-Aminogruppen aufweisenden Verbindungen bevorzugt stöchiometrische Mengen (bezogen auf die Menge der Harnstoffgruppen eingesetzt) an Base eingesetzt. Ein kleiner Überschuss der Base kann zur Sicherheit eingesetzt werden, ein grösserer Überschuss bringt keinerlei Vorteile. Die Hydrolyse wird bei einer Innentemperatur von etwa 40 bis 100°C, bevorzugt 80 bis 100°C, durchgeführt, sie ist bei der Verwendung von stöchiometrischen Mengen an Base i.a. nach 15 Minuten bis 6 Stunden, zumeist 30 Minuten bis 2 Stunden, beendet.

Bei Verwendung katalytischer (0,001 bis 0,1 Äquivalente an t-Amin auf 1 Äquivalent Harnstoffgruppen) und unterstöchiometrischen Mengen an t-Aminogruppen aufweisenden Verbindungen (z.B. 0,1 bis 0,5 Äquivalente an t-Aminogruppen auf 1 Äquivalent Harnstoffgruppen) dauert die Reaktion bis zu 12 d. Dabei entweicht $CO_2$.

Nach Beendigung der Reaktion wird im allgemeinen so aufgearbeitet, dass durch Destillation, die bevorzugt bei vermindertem Druck, z.B. 13 bis 900 hPa, durchgeführt wird, das bei der Hydrolyse angefallene sekundäre Amin, Wasser und/oder gegebenenfalls vorhandenes tert.-Amin abdestilliert und bevorzugt für eine weitere Verwendung aufbereitet werden. Nachdem von etwaigen Feststoffen (z.B. Na-, K-(Hydrogen)carbonate) abfiltriert wurde, ist die Aufarbeitung beendet.

Hydrolyse und Aufarbeitung können auch so kombiniert werden, dass während der Hydrolyse das entweichende sekundäre Amin abdestilliert wird. Dies ist selbstverständlich jedoch nur dann möglich, wenn zwischen dem sekundären Amin einerseits und Wasser/t-Amingemisch andererseits eine destillative Trennung in befriedigendem Umfang gelingt.

Das Ende der Reaktion bzw. die vollständige Umwandlung der Harnstoffgruppen in Amineinheiten kann z.B. an der Menge des sek. Amins, an der Menge des entweichenden Kohlendioxyds oder am Verschwinden der Harnstoffbande im IR-Spektrum beobachtet werden.

Variante B (allgemeine Verfahrensweise)

Bei dieser Modifikation des erfindungsgemässen Verfahrens wird der zu hydrolisierende Harnstoff in situ erzeugt. Dabei wird sich zunutze gemacht, dass aromatische Isocyanate mit sekundären Aminen bei 20 bis 100°C im allgemeinen schneller als mit Wasser zu Harnstoffen oder Hydroxidgruppen zu Carbamaten reagieren. Es muss in dieser Verfahrensvariante jedoch gewährleistet sein, dass stets ausreichend sekundäres Amin zur Reaktion mit den NCO-Gruppen der NCO-Verbindung vorhanden ist. Eine ausreichende Menge an sekundärem Amin kann dadurch gewährleistet sein, dass 1. eine stöchiometrische Menge an sekundärem Amin vorgelegt wurde oder das 2. die Harnstoffbildung unter solchen Bedingungen erfolgt, dass alsbaldige Hydrolyse dieser Harnstoffverbindung unter Freisetzung des sekundären Amins, das wiederum zur erneuten Harnstoffbildung mit weiteren Isocyanaten verwendet wird, erfolgt.

1. Verwendung stöchiometrischer Mengen an sekundärem Amin (Äquivalentverhältnis NH:NCO$\geq$1)

Im erfindungsgemässen Verfahren wird das NCO-Prepolymer in der Regel ohne Lösungsmittel verwendet. Es können jedoch nur z.B. Viskositätserniedrigung auch Lösungen von NCO-Prepolymeren in NCO-inerten Lösungsmitteln verwendet werden, die vorzugsweise auch wassermischbar sind. Geeignete Lösungsmittel in diesem Sinne sind z.B. Dimethoxyethan, Diethylenglykoldimethylether, Dioxan oder Tetrahydrofuran, weniger geeignete Lösungsmittel in diesem Sinne sind z.B. Kohlenwasserstoffe, chlorierte Kohlenwasserstoffe sowie niedere Aromaten, chlorierte und nitrierte Aromaten. Bevorzugt werden die NCO-Prepolymere jedoch dann als Lösungen in den obengenannten Lösungsmitteln verwendet, wenn es sich um feste oder im Bereich von 20 bis 80°C unschmelzbare, schwerschmelzbare oder zähflüssige NCO-Prepolymere handelt.

Werden flüssige, reine, nicht gelöste NCO-Prepolymere im erfindungsgemässen Verfahren eingesetzt, haben sie vorteilhafterweise eine Temperatur von 20 bis 80°C, bevorzugt von 40 bis 70°C. Werden die NCO-Prepolymere in gelöster Form eingesetzt, so beträgt die bevorzugte Temperatur 20 bis 40°C, höchstens jedoch die dem Siedepunkt des betreffenden Lösungsmittels entsprechende Temperatur. Werden die NCO-Prepolymere als Lösungen verwendet, so können z.B. auf 100 Teile Lösungsmittel 1 bis 400 Teile NCO-Prepolymer eingesetzt werden.

Die NCO-Verbindungen werden mit einem auf 0 bsi 60°C temperierten basischen Medium vermischt.

Dieses basische Medium enthält:

1. Wasser, und zwar bevorzugt $\geq$1 Teil Wasser auf 1 Teil NCO-Verbindung. Das basische Medium enthält ferner

2. $OH^{\ominus}$-Gruppen besitzende Basen und/oder tert.-Aminogruppen aufweisende Verbindungen;

3. ein sekundäres aliphatisches Amin sowie gegebenenfalls

4. ein Lösungsmittel.

Als $OH^{\ominus}$-Gruppen besitzende Basen sind organische und anorganische Hydroxidgruppen enthaltende Verbindungen geeignet.

Als organische Basen seien die Tetraalkylammoniumhydroxide und auf einem unlöslichen Polymerskelett Trialkylammoniumhydroxid-Reste fixiert enthaltende basische Ionenaustauscher genannt, bevorzugt sind jedoch die anorganischen Alkali- und Erdalkalihydroxide, besonders bevorzugt sind Natrium- und Kaliumhydroxid.

Die $OH^{\ominus}$-Gruppen besitzenden Basen werden in solchen Mengen eingesetzt, dass auf eine NCO-Gruppe (bezogen auf eine sich im Verlauf der Reaktion mit dem sekundären Amin bildende 1-Aryl-3,3-dialkyl-Harnstoffgruppe) 0 bis 2 $OH^{\ominus}$-Gruppen kommen, bevorzugt 0,1 bis 1,8, besonders bevorzugt 1,00 bis 1,5.

Werden keine $OH^{\ominus}$-Gruppen besitzende Basen eingesetzt, so müssen t-Aminogruppen aufweisende Verbindungen zugegen sein.

Werden die $OH^{\ominus}$-Gruppen besitzenden Basen in einer solchen Menge verwendet, dass auf eine NCO-Gruppe 0,1 bis 0,99 $OH^{\ominus}$-Gruppen kommen, so wird eine weitere t-Amingruppen aufweisende Verbindung mitverwendet. Die Verwendung der Basen im Verhältnis $OH^{\ominus}$:NCO von 0,1 bis 0,99 ist aber insgesamt weniger bevorzugt, da dann die Harnstoffhydrolyse, insbesondere im unteren Basen-Konzentrationsbereich, sehr langsam erfolgt und ohnehin ein Filtrationsschritt nötig ist. Bevorzugt ist die Verwendung einer der Menge der 1-Aryl-3,3-dialkylharnstoff-Gruppen äquivalenten oder leicht überschüssigen Menge an $OH^{\ominus}$-Gruppen aufweisenden Basen.

Das basische Medium kann ferner tert.-Aminogruppen aufweisende Verbindungen enthalten. Sie können alternativ zu den $OH^{\ominus}$-Gruppen aufweisenden Basen verwendet werden, doch können sie mit jenen auch in einem beliebigen Gemisch eingesetzt werden. Es sind die gleichen Mengenbereiche wie bei der Verwendung von $OH^{\ominus}$-Ionen enthaltenden Basen bevorzugt, wobei sich die Mengenangaben auf die Menge der t-Aminogruppen beziehen. Geeignete, t-Aminogruppen aufweisende Verbindungen wurden bereits aufgeführt.

Die t-Aminogruppen aufweisenden Verbindungen können in reiner (flüssiger oder fester) oder gelöster Form zugegeben werden. Bevorzugtes Lösungsmittel ist Wasser, verwendbar sind ferner Lösungsmittel, die weder mit NCO-Gruppen noch mit sekundären Aminen reagieren und wasserlöslich sind. Geeignete Lösungsmittel in diesem Sinne sind (ausser Wasser) etwa Dioxan, Tetrahydrofuran, Ethandiol-1,2-dimethylether, 3-Oxa-pentan-1,5-diol-dimethylether (Diglyme). Es können z.B. auf 1 Teil einer t-Aminogruppen aufweisenden Verbindung 0,5 bis 100 Teile eines Solvens verwendet werden.

Allgemein ist die Verwendung von $OH^{\ominus}$-Gruppen besitzenden Basen gegenüber der Verwendung von t-Aminogruppen aufweisenden Verbindungen und gegenüber der Verwendung von Gemischen aus t-Aminogruppen aufweisenden Verbindungen und $OH^{\ominus}$-Gruppen besitzenden Basen bevorzugt.

Geeignete sekundäre Amine wurden bereits aufgezeigt. Für das erfindungsgemässe Verfahren gemäss dieser Verfahrensvariante besonders geeignete sekundäre Amine sind relativ leicht flüchtige Amine wie Dimethylamin, Diethylamin, Di-n-propylamin, Diethylamin, Di-n-propylamin, Di-i-propylamin, Di-n-butylamin, Di-i-butylamin und Di-sec.-butylamin, ganz besonders geeignet und bevorzugt ist Dimethylamin.

Die sekundären Amine wurden in dieser Verfahrensvariante, bezogen auf die Menge der NCO-Gruppen in stöchiometrischen Mengen oder in einem Überschuss eingesetzt (NCO:NH = 1:1–1:1.1), doch ist auch ein grosser Überschuss (NCO:sec.-Amin = 1:1 bis 1:10) verwendbar, wobei das sekundäre Amin dann als Verdünnerkomponente fungiert.

Zur Viskositätserniedrigung des Gemisches kann vor der Zugabe des NCO-Prepolymeren oder während jedes beliebigen Zeitpunkts der Reaktion ein weiteres Lösungsmittel, das vorzugsweise mit Wasser mischbar ist, zugegeben werden, z.B. Dioxan, Tetrahydrofuran und Dimethoxyethan. Zum Beispiel können auf 100 Teile Wasser 10 bis 1000 Teile Cosolvens mitverwendet werden.

Die NCO-Prepolymere werden im erfindungsgemässen Verfahren langsam mit dem basischen Medium vereinigt. Dies kann mittels eines Tropftrichters oder durch z.B. maschinelle Einspritzung mit einer Düse oder auch z.B. durch Gegenstrominjektion geschehen.

Die Zeitdauer der Vereinigung mit dem NCO-Prepolymeren richtet sich nach der Wärmetönung der Reaktion, wobei durch Kühlung dafür Sorge getragen werden muss, dass die Temperatur im Reaktionsgefäss nicht die oben angeführten Temperaturschranken übersteigt. Dies ist besonders wichtig im Falle von tri- und höherfunktionellen NCO-Prepolymeren und bei nicht-kontinuierlicher Verfahrensweise.

Nachdem alles NCO-Prepolymer zugegeben wurde, kann weiteres Lösungsmittel zugefügt werden, obwohl dies im allgemeinen nicht nötig ist. Ausser den oben angeführten Solventien sind auch z.B. wasserlösliche Alkohole wie Methanol, Ethanol, i-Propanol, Glykolmonomethylether, ferner z.B. Acetonitril, Nitromethan brauchbar, weniger geeignet sind wassernichtmischbare Solventien wie Ether, Cyclohexan, Toluol etc.

a) Verfahrensvariante A

Die resultierende Suspension und/oder Lösung des Harnstoffs wird nun auf eine Temperatur gebracht, bei der Rückfluss eintritt, z.B. 80 bis 100°C. Das Reaktionsgemisch wird 3 h bis 10 d, bevorzugt 6 h bis 2 d, zum Rückfluss erhitzt, und durch fraktionierte Destillation von allen flüchtigen Bestandteilen befreit. Aus dem Destillat kann das sekundäre Amin und/oder die tertiäre Aminogruppen aufweisende Verbindung nach an sich bekannten Methoden gewonnen werden. Diese Destillation kann bei Normaldruck oder

auch bei vermindertem Druck, z.B. 13 bis 910 hPa, vorgenommen werden. Zur Entfernung letzter Spuren flüchtiger Bestandteile wird zweckmässigerweise nochmals bei erhöhter Temperatur (80 bis 100°C) Ölpumpenvakuum (0,13 bis 7 hPa) angelegt.

Vom noch warmen (40 bis 100°C) Destillationsrückstand werden etwaig ausgefallene, z.B. Kohlensäuresalze, abfiltiert bzw. abgesaugt. Vorzugsweise geschieht dies mit einer gegebenenfalls beheizbaren Drucknutsche.

b) Verfahrensvariante B

Bevorzugt wird mit dem Harnstoffreaktionsgemisch so verfahren, dass nicht zum Rückflus erhitzt wird, sondern die flüchtigen Bestandteile, insbesondere das abgespaltene sekundäre Amin, während der Reaktion abdestilliert werden. An der aufgefangenen Menge an sekundärem Amin kann gegebenenfalls auch der Fortgang der Reaktion kontrolliert werden. Diese Variation ist besonders beim Vorliegen der Dimethyl- und Diethylamin-Harnstoff-Verbindungen geeignet, bei anderen sec.-Amin-Harnstoffkomponenten muss u.U. während der Reaktion Lösungsmittel (Wasser) zur Gewährleistung der Rührfähigkeit nachgegeben werden. Diese Variante ist auch dann besonders geeignet, wenn ausschliesslich nichtflüchtige Alkalihydroxide als Basen verwendet werden, während t-Aminogruppen aufweisende Verbindungen u.U. mit abdestilliert werden.

Diese Variante wird bevorzugt bei 80 bis 100°C und ohne Anwendung von Vakuum durchgeführt. Nach Abdestillation des sekundären Amins bei Normaldruck werden die anderen flüchtigen Komponenten bei Normaldruck oder vermindertem Druck bei z.B. 13,3 bis 933 hPa und dann z.B. 0,13 bis 13 hPa und Temperaturen von 40 bis 100°C abdestilliert.

Danach wird vom noch warmen (40 bis 100°C) Destillationsrückstand das Kohlensäuresalz abfiltriert oder mit einer (bevorzugt beheizten) Drucknutsche abgesaugt.

Bei sehr hohen Produktviskositäten kann es notwendig sein, vor dem Absaugen das Kohlensäuresalz mit einem inerten Lösungsmittel wie Dichlormethan oder Toluol zu verdünnen. Dieses Lösungsmittel wird nach dem Absaugen wieder destillativ entfernt.

2. Verwendung unterstöchiometrischer Mengen an sekundärem Amin (Äquivalentverhältnis NH:NCO < 1)

Auch in dieser Modifikation des erfindungsgemässen Verfahrens wird das NCO-Prepolymer in der Regel ohne Lösungsmittel verwendet. Es können jedoch zur z.B. Viskositätserniedrigung auch Lösungen von NCO-Prepolymeren in NCO-inerten Lösungsmitteln verwendet werden, die vorzugsweise auch wassermischbar sind. Geeignete Lösungsmittel in diesem Sinne sind z.B. Dimethoxyethan, Diethylenglykoldimethylether, Dioxan oder Tetrahydrofuran, weniger geeignete Lösungsmittel in diesem Sinne sind z.B. Kohlenwasserstoffe, chlorierte Kohlenwasserstoffe sowie niedere Aromaten, chlorierte und nitrierte Aromaten. Bevorzugt werden die NCO-Prepolymere jedoch dann als Lösungen in den obengenannten Lösungsmitteln verwendet, wenn es sich um feste oder im Bereich von 20 bis 80°C unschmelzbare, schwerschmelzbare oder zähflüssige NCO-Prepolymere handelt.

Werden flüssige, reine, nicht gelöste NCO-Prepolymere im erfindungsgemässen Verfahren eingesetzt, haben sie vorteilhafterweise eine Temperatur von 20 bis 80°C, bevorzugt von 40 bis 70°C. Werden die NCO-Prepolymere in gelöster Form eingesetzt, so beträgt die bevorzugte Temperatur 20 bis 40°C, höchstens jedoch die dem Siedepunkt des betreffenden Lösungsmittels entsprechende Temperatur. Werden die NCO-Prepolymere als Lösungen verwendet, so können z.B. auf 100 Teile Lösungsmittel 1 bis 400 Teile NCO-Prepolymer eingesetzt werden.

Die NCO-Verbindungen werden mit einem auf 60 bis 100°C, bevorzugt 80 bis 100°C, heissen basischen Medium vermischt, das unter Rückfluss gehalten wird.

Dieses basische Medium enthält:

1. Wasser, und zwar bevorzugt ≧1 Teil Wasser auf 1 Teil NCO-Verbindung;
2. $OH^{\ominus}$-Gruppen enthaltende Basen und/oder t-Aminogruppen aufweisende Verbindungen;
3. ein sekundäres aliphatisches Amin sowie gegebenenfalls
4. ein Lösungsmittel.

Die $OH^{\ominus}$-Gruppen enthaltenden Basen und die t-Aminogruppen aufweisenden Verbindungen, die in dieser Variante des erfindungsgemässen Verfahrens eingesetzt werden, wurden bereits aufgeführt.

Sie werden bevorzugt in stöchiometrischen Mengen (1 mol $OH^{\ominus}$ bzw. 1 mol an t-Aminogruppen auf 1 mol NCO) oder geringfügig überschüssigen Mengen verwendet. $OH^{\ominus}$-Gruppen enthaltende Basen sind auch hier gegenüber t-Aminogruppen aufweisenden Verbindungen bevorzugt.

Die in dieser Variante verwendbaren sekundären Amine sind dieselben, wie sie bereits aufgeführt wurden.

Aus verfahrenstechnischen Gründen sind Dimethylamin und Diethylamin hier weniger bevorzugt. Die vorzugsweise nicht allzu leicht flüchtigen sekundären Amine werden aber hier in solchen Mengen eingesetzt, dass auf eine NCO-Gruppe aus der NCO-Verbindung 0,05 bis 0,99, bevorzugt 0,3 bis 0,6 sek.-Aminogruppen treffen.

Zur Viskositätserniedrigung kann vor der Vereinigung mit der NCO-Komponente bzw. zu jedem beliebigen Zeitpunkt der Reaktion ein vorzugsweise wassermischbares, NCO-nichtreaktives Lösungsmittel wie z.B. Dioxan, Tetrahydrofuran und Dimethoxyethan zugegeben werden. Zum Beispiel können auf 100 Teile Wasser 10 bis 1000 Teile eines weiteren Lösungsmittels oder Lösungsmittelgemischs zugegeben werden.

Die Reaktion wird im allgemeinen so vorgenommen, dass der Wasser, Basen und sekundäres Amin enthaltende Reaktionsraum auf 20 bis 100°C, bevorzugt 80 bis 100°C, besonders bevorzugt 100°C, erhitzt und während der gesamten Reaktionsdauer auf dieser Temperatur gehalten wird. Diese Reaktionsmischung wird nun mit einer solchen Menge der NCO-Verbindung vereinigt, dass nach Reaktion der NCO-Gruppen mit allen sekundären Aminmolekülen keine freien NCO-Gruppen zugegen sind. Durch die im Reaktionsraum vorliegenden Temperaturen und die Base wurden die entstandenen Harnstoffgruppen wieder gespalten. Hierbei entsteht das gewünschte aromatische Amin und das ursprüngliche sekundäre Amin, das mit weiteren NCO-Verbindungen erneut in Reaktion treten kann.

Die Vereinigung der Basen enthaltenden Mischung mit der NCO-Komponente geschieht in dem Masse, wie aus dem Harnstoff sekundäres Amin abgespalten wird und als Reaktionspartner zur Verfügung steht.

Die nötige Reaktionszeit hängt ab von der Konzentration des sekundären Amins, der Reaktionstemperatur und der Abspaltbarkeit des sekundären Amins aus dem Harnstoff. Im allgemeinen werden 2 h bis 2 d, bevorzugt 6 h bis 18 h zur vollständigen Reaktion benötigt.

Nach Beendigung der Reaktion wird das sekundäre Amin und das Lösungsmittel und Wasser bei z.B. 100°C/13,3 bis 900 hPa und 100°C/0,13 bis 13 hPa abdestilliert und evtl. angefallenes Kohlensäuresalz abfiltriert. Bei sehr hohen Produktviskositäten kann es vorteilhaft sein, mit einem Verdünner wie Dichlormethan oder Toluol die Viskosität abzusenken. Wird dies nicht getan, so erfolgt das Absaugen bzw. Abfiltrieren des Kohlensäuresalzes von 60 bis 100°C warmen Produkt. Das Abfiltrieren wird vorzugsweise mit einer bezeizbaren Drucknutsche vorgenommen.

Die erfindungsgemäss erhaltenen Polyamine stellen mittelviskose bis hochschmelzende Produkte dar. Sie haben einen Gehalt an Gesamtstickstoff von ca. 38% bis 0,5%, bevorzugt 20% bis 0,8% und besonders bevorzugt von 4,3% bis 1,2%. Ihr Gehalt an primärem Stickstoff beträgt 19% bis 0,25%, bevorzugt 10% bis 0,4%, besonders bevorzugt 2,15% bis 0,6%.

Die erfindungsgemäss erhaltenen Polyamine werden wegen ihres niedrigen Dampfdrucks vorzugsweise als Reaktionspartner für Polyisocyanate bei der Herstellung von gegebenenfalls zelligen Polyurethankunststoffen eingesetzt, wobei sie gegebenenfalls auch mit anderen niedermolekularen (Molekulargewicht: 32 bis 399) und/oder höhermolekularen (Molekulargewicht: 400 bis ca. 12 000) Verbindungen mit gegenüber Isocyanaten reaktiven Gruppen kombiniert werden können. Geeignete Ausgangskomponenten für die Herstellung von Polyurethankunststoffen werden oben im Zusammenhang mit der Prepolymerherstellung bzw. auch in DE-A 2 302 564, DE-A 2 432 764 (US-PS 3 963 679) sowie in den DE-A 2 639 083, 2 512 385, 2 513 815, 2 550 796, 2 550 797, 2 550 833, 2 550 860 und 2 550 862 genannt. Dort finden sich auch Hinweise auf bei der Polyurethanherstellung gegebenenfalls mitzuverwendende Hilfs- und Zusatzstoffe. Die Herstellung von Polyurethanharnstoffen mittels der erfindungsgemäss hergestellten Polyamine ist ebenfalls Gegenstand der vorliegenden Erfindung.

Weitere Verwendungszwecke der erfindungsgemäss hergestellten Polyamine sind z.B. Kupplungskomponenten für Diazofarbstoffe, Härter für Epoxid- und Phenolharze sowie alle anderen an sich bekannten Reaktionen von Aminen wie Amid- oder Imidbildung etc.

Die folgenden Beispiele dienen der Erläuterung des erfindungsgemässen Verfahrens. Wenn nicht anders vermerkt, sind Mengenangaben als Gewichtsteile bzw. Gewichtsprozente zu verstehen.

Beispiele

Die Beispiele 1 und 2 fallen nicht unter den Anspruchsumfang, sie demonstrieren aber die Effizienz der anmeldungsgemässen Verfahrensschritte.

Beispiel 1

Beständigkeit von 1-Aryl- und 1-Alkyl-3,3-dialkyl-, -3-alkyl-, -3,3-diaryl- und -3-arylharnstoffen gegenüber basischer Hydrolyse

Je 100 g der aus Phenylisocyanat bzw. Methylisocyanat und verschiedenen Aminen hergestellten Harnstoffverbindungen wurden mit 10 g Wasser und 20 g Natriumhydroxid 3 Stunden auf 100°C erhitzt. Etwaiges Destillat wurde gaschromatographisch auf den Gehalt an abgespaltenem Amin untersucht.

Folgende Ergebnisse wurden erhalten:

| Harnstoff | abgespaltenes Amin (Sdp.) | (°C) | Aminausbeute nach 3 h (%) |
|---|---|---|---|
| 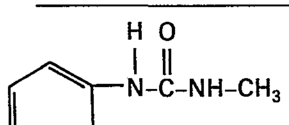 | $H_2NCH_3$ [1] | (-6) | 0 [2] (nicht erfindungsgemäss) |
| 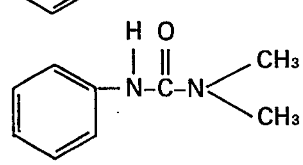 | $NH(CH_3)_2$ | (7,4) | 98 [2] (erfindungsgemäss) |

| Harnstoff | abgespaltenes Amin (Sdp.) (°C) | Aminausbeute nach 3 h (%) |
|---|---|---|
| 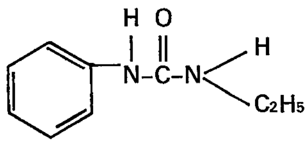 | $H_2NC_2H_5$ 1) (17) | 0 2) (nicht erfindungsgemäss) |
| 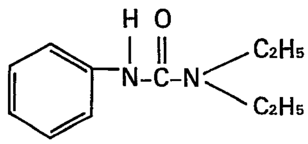 | $HN(C_2H_5)_2$ (56) | 84 (erfindungsgemäss) |
| 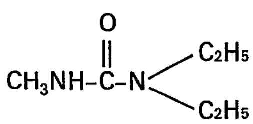 | $HN(C_2H_5)_2$ (56) | 0 (nicht erfindungsgemäss) |

1) hergestellt aus Methylisocyanat resp. Ethylisocyanat und Anilin
2) Direkte Einleitung des gasförmigen Destillats in verdünnte Salzsäure und Rücktitration der überschüssigen Säure.

Beispiel 2
Beständigkeit von aus Diisocyanaten und aromatischen und aliphatischen sekundären Aminen gewonnenen Harnstoffen gegenüber basischer Hydrolyse

2.1.
Je 100 g der aus Toluylendiisocyanat bzw. Hexan-1,6-diisocyanat und verschiedenen Aminen hergestellten Harnstoffverbindungen werden mit 20 g Natriumhydroxid und 40 g Wasser 3 Stunden auf 100°C erhitzt. Etwaiges Destillat wurde titrimetrisch oder gaschromatographisch auf den Gehalt an abgespaltenem Amin untersucht.
Folgende Ergebnisse wurden erhalten:

| Harnstoff | abgespaltenes Amin (Sdp.) | (°C) | Aminausbeute nach 3 h/100°C (%) |
|---|---|---|---|
| 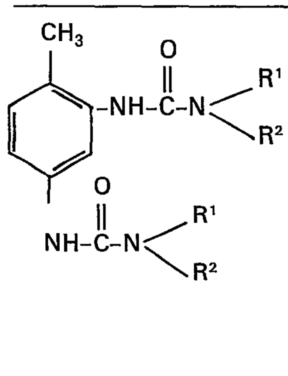 | | | |
| (aromatischer Rest/erfindungsgemäss) | | | |
| | primäre Amine | | (nicht erfindungsgemäss) |
| $R^1=H, R^2=CH_3$ 1) | $H_2NCH_3$ | (-6) | 0 2) |
| $R^1=H, R^2=C_2H_5$ 1) | $H_2NC_2H_5$ | (17) | 0 2) |
| $R^1=H, R^2=C_3H_7$ | $H_2NC_3H_7$ | (49) | 0 2) |
| $R^1=H, R^2=i-C_3H_7$ | $H_2N-i-C_3H_7$ | (32) | 0 2) |

| Harnstoff | abgespaltenes Amin (Sdp.) | (°C) | Aminausbeute nach 3 h/100°C (%) |
|---|---|---|---|
| 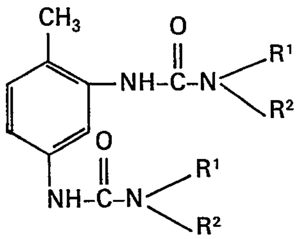 | | | |
| | sekundäre Amine | | (erfindungsgemäss) |
| $R^1=R^2=CH_3$ | $HN(CH_3)_2$ | (7,4) | 94% |
| $R^1=R^2=C_2H_5$ | $HN(C_2H_5)_2$ | (56) | 78% |
| 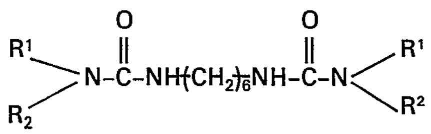 | | | |
| aliphatisches Diisocyanat (nicht erfindungsgemäss) | | | |
| $R^1=H, R^2=CH_3$ 1) | $H_2NCH_3$ | (-6) | 0 2) |
| $R^1=H, R^2=C_2H_5$ 1) | $H_2NC_2H_5$ | (17) | 0 2) |
| $R^1=H, R^2=i\text{-}C_3H_7$ | $H_2NiC_3H_7$ | (32) | 0 2) |
| $R^1=R^2=C_2H_5$ | $HN(C_2H_8)_2$ | (56) | 0 |

1) hergestellt aus Methylisocyanat resp. Ethylisocyanat und 2,4-Diaminotoluol
2) Direkte Einleitung des gasförmigen Kondensats in verdünnte Salzsäure und Rücktitration der überschüssigen Säuren

2.2.

Je 100 g des aus Toluylendiisocyanat bzw. Hexan-1,6-diisocyanat mit verschiedenen primären und sekundären Aminen hergestellten Harnstoffverbindungen wurden mit 20 g Natriumhydroxid und 60 g Wasser zu Rückfluss erhitzt. Nach 3stündigem Kochen am Rückfluss wurden alle flüchtigen Substanzen bei 100°C/21 hPa abdestilliert und der Gehalt an abgespaltenem Amin bestimmt. Danach wird erneut 60 ml Wasser hinzugefügt, erneut 3 Stunden am Rückfluss gehalten und erneut abdestilliert und der Gehalt an abgespaltenem Amin bestimmt.

Folgende Ergebnisse wurden erhalten:

| Harnstoff | abgespaltenes Amin (Sdp.) | (°C) | Aminausbeute nach 3 h | nach 6 h (%) |
|---|---|---|---|---|
| 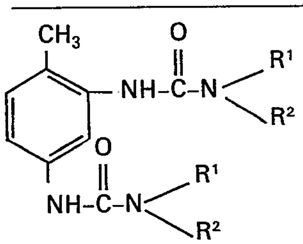 | | | | |
| $R^1=H, R^2=$ Cyclohexyl | 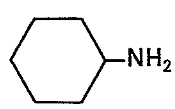 | (134,5) | 0 | 0 (nicht erfindungsgemäss) |

| Harnstoff | abgespaltenes Amin (Sdp.) | (°C) | Aminausbeute nach 3 h/nach 6 h (%) | |
|---|---|---|---|---|
| $R^1=H, R^2=C_2H_5$ | 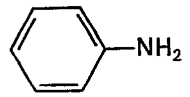 | (184) | 1 | 3 (nicht erfindungsgemäss) |
| $R^1=R^2=n\text{-}C_4H_9$ | $HN(n\text{-}C_4H_8)_2$ | (19) | 63 | 81 (erfindungsgemäss) |

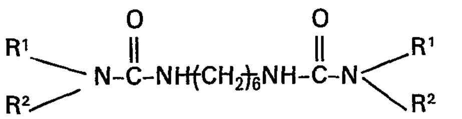

aliphatische Diisocyanat-Basis (nicht erfindungsgemäss)

| | | | | |
|---|---|---|---|---|
| $R^1=H, R^2=$Cyclohexyl | 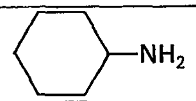 | (134,5) | 0 | 0 |
| $R^1=H, R^2=C_6H_5$ | 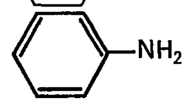 | (184) | 0 | 0 |
| $R^1=R^2=n\text{-}C_4H_9$ | $HN(C_4H_9)_2$ | (159) | 0 | 0 (nicht erfindungsgemäss) |

Beispiel 3

Herstellung eines linearen Diaminopolyethers

3.1. Herstellung des Harnstoffs

1500 g eines aus Toluylen-2,4-diisocyanat und einem Polypropylenglykoletherdiol der durchschnittlichen Molmasse 2000 hergestellten NCO-Prepolymers mit einem NCO-Gehalt von 3,6% (1,284 mol NCO) werden in 750 ml wasserfreiem Dioxan gelöst. Innerhalb 30 Minuten werden bei einer 43°C nicht übersteigenden Temperatur 93,75 g (1,284 mol) Diethylamin zugegeben. Die klare Lösung, die IR-spektroskopisch keine freien NCO-Gruppen mehr aufweist, wird 15 Minuten nachgerührt.

3.2. Herstellung des Amins

Der obige Reaktionsansatz wurde in drei gleiche Teile geteilt und nach verschiedenen Methoden aufgearbeitet.

3.2.1. Verwendung einer äquimolaren Menge Kaliumhydroxid

761 g der obigen Reaktionsmischung, die 425 mmol Harnstoffgruppen aufweist, werden bei 20°C mit 48 g 50%iger Kalilauge (enthält 428 mmol KOH) versetzt, worauf sofortige Verfärbung nach Gelb auftritt. Die Reaktionsmischung wurde nun 9 Stunden auf 100°C erhitzt, wobei ein Diethylamin enthaltendes wässriges Azeotrop überging. Durch titrimetrische Untersuchung des Kondensats auf Amin wurde das Ende der Reaktion festgestellt und bei 100°C/26 hPa die flüchtigen Bestandteile des Reaktionsgemischs abdestilliert. Das 80°C warme Reaktionsprodukt wurde durch Absaugen vom ausgefallenen Kohlensäuresalz befreit. Insgesamt wurden 30,3 g (97% der Theorie) Diethylamin zurückerhalten.

Daten in Tabelle 1.

3.2.2. Verwendung einer äquimolaren Menge an t-Aminogruppen enthaltenden Verbindungen (Diazabicyclooctan, «Dabco»)

761 g der obigen Reaktionsmischung werden bei 20°C mit 24 g Wasser und 24 g Diazabicyclooctan (214 mmol ≙ 428 mmol t-Aminogruppen) versetzt und so lange auf 100°C erhitzt, bis im Kondensat kein Diethylamin mehr nachzuweisen ist (insgesamt 20 Stunden).

Aufarbeitung wie unter 3.2.1., Daten in Tabelle 1.

3.2.3. Verwendung einer unterstöchiometrischen Menge an einer tert.-Aminogruppen aufweisenden Verbindung

761 g der obigen Reaktionsmischung wurden bei 20°C mit 24 g Wasser und 2,4 g Diazabicyclooctan (21,4 mmol) versetzt und so lange auf 100°C erhitzt, bis im Kondensat kein Diethylamin mehr nachzuweisen ist (insgesamt 72 Stunden). Aufarbeitung wie unter 3.2.1., Daten in Tabelle 1.

Tabelle 1

Daten der Versuche 3.2.1., 3.2.2. und 3.2.3.

| Versuch | 3.2.1. | 3.2.2. | 3.2.3. |
|---|---|---|---|
| Ausbeute (%) | 96 | 96 | 89 |
| NH-Zahl (mg KOH/g) | 43,4 | 40,0 | 38,8 |
| Säurezahl (mg KOH/g) | <0,05 | <0,05 | <0,05 |

Tabelle 1 (Fortsetzung)
Daten der Versuche 3.2.1., 3.2.2. und 3.2.3.

| Versuch | 3.2.1. | 3.2.2. | 3.2.3. |
|---|---|---|---|
| Molmasse | 2900 | 3200 | 3400 |
| Viskosität (mPa.s) (bei 75°C) | 430 | 510 | 590 |
| Wassergehalt (%) (Karl Fischer) | 0,11 | 0,08 | 0,08 |

Beispiel 4
Herstellung eines linearen Diaminopolyethers

4.1. Herstellung des Harnstoffs
1000 g eines Prepolymers, das einen NCO-Gehalt von 3,5% aufweist und durch Umsetzung von Toluylendiisocyanat mit einem Polypropylenglykoletherdiol der durchschnittlichen Molmasse 2000 im NCO:OH-Verhältnis von 2:1 hergestellt wurde, wird bei Raumtemperatur in exothermer Reaktion mit 118,3 g Diethylamin (1,62 mol) zum entsprechenden flüssigen Harnstoff umgesetzt.

4.2. Herstellung des Amins
Nach 10minütigem Nachrühren wird mit 72 ml 45%iger Natronlauge verdünnt und 6,5 Stunden auf 100°C ohne Abdestillation flüchtiger Komponenten erhitzt.
Die resultierende, von Kristallen durchsetzte Masse wird mit 500 ml Toluol versetzt und die Kristalle durch Absaugen abgetrennt (Natriumcarbonat resp. Hydrogencarbonat). Das toluolische Filtrat wird bei 100°C/18 hPa und dann 100°C/1,33 hPa von allen flüchtigen Verbindungen befreit.

Produktdaten:

| | |
|---|---|
| Ausbeute (%) | 97 |
| NH-Zahl (mg KOH/g) | 44,1 |
| Säurezahl (mg KOH/g) | <0,05 |
| Molmasse | 2600 |
| Viskosität (mPa.s) (bei 75°C) | 445 |
| Wassergehalt (%) (Karl Fischer) | 0,1 |

Beispiel 5
Herstellung eines trifunktionellen Polyethers

5.1. Herstellung des Harnstoffs
Vorgelegt werden 1000 g eines NCO-Prepolymers (NCO-Gehalt 1,93%), das hergestellt wurde aus Toluylen-2,4-diisocyanat und einem auf Trimethylolpropan gestarteten Polypropylenoxid/Polyethylenoxid-Blockcopolyethertriol der durchschnittlichen Molmasse 6000 im NCO:OH-Verhältnis 2:1. Innerhalb 30 Minuten wird mit 40,99 g 50,5%iger wässriger Dimethylaminlösung (enthält 0,46 mol Dimethylamin) der entsprechende flüssige Harnstoff hergestellt.

5.2. Herstellung des Amins
Nach 15minütigem Nachrühren wird obiger Reaktionsansatz mit 40,9 ml 45%iger Natronlauge versetzt und 3 Stunden auf 100°C erhitzt. Dabei entweicht Dimethylamin gasförmig. Das resultierende Produktgemisch wird bei 100°C/18 h Pa und 100°C/1,33 hPa vom Wasser befreit und aus der auf 80°C abgekühlten öligen Flüssigkeit mit einer Drucknutsche das Kohlensäuresalz abgetrennt.

Produktdaten:

| | |
|---|---|
| Ausbeute (%) | 93 |
| NH-Zahl (mg KOH/g) | 22 |
| Säurezahl (mg KOH/g) | 0,05 |
| Molmasse | 7000 |
| Viskosität (mPa.s) (bei 75°C) | 710 |
| Wassergehalt (%) (Karl Fischer) | 0,17 |

Beispiel 6
Herstellung eines linearen Aminopolyethers unter Verwendung unterstöchiometrischer Mengen an sekundärem Amin
1000 g des NCO-Prepolymers aus Beispiel 3 (NCO-Gehalt 3,6%, 856 mmol NCO) werden bei 100°C zu einer vorgelegten Mischung aus 100 g Wasser, 12,9 g Dibutylamin (100 mmol) und 48 g KOH (856 mmol) mit einer gleichbleibenden Geschwindigkeit von 120 g NCO-Prepolymer/h zugegeben. Nach Beendigung wird 2 Stunden bei dieser Temperatur weitergerührt und dann bei 20 hPa die flüchtigen Bestandteile abdestilliert. Der ölige Rückstand wird mit 800 ml Dichlormethan aufgenommen, durch Absaugen vom Kohlensäuresalz befreit und erneut bei 80°C/18 hPa und dann 100°C/1,33 hPa vom Lösungsmittel befreit.

Produktdaten:

| | |
|---|---|
| Ausbeute (%) | 86 |
| NH-Zahl (mg KOH/g) | 47,4 |
| Säurezahl (mg KOH/g) | 0,05 |
| Molmasse | 2400 |
| Viskosität (mPa.s) (bei 75°C) | 435 |
| Wassergehalt (%) (Karl Fischer) | 0,06 |

**Patentansprüche**

1. Verfahren zur Herstellung von Urethan- und/oder Harnstoff- und/oder Allophanat- und/oder Biuret- und/oder Isocyanurat- sowie bevorzugt auch Ethergruppen enthaltenden aromatischen Polyaminen, dadurch gekennzeichnet, dass eine Verbindung der allgemeinen Formel

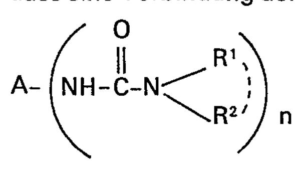

wobei
A ein Rest ist, wie er durch Entfernung von n Isocyanatgruppen aus einer Urethan- und/oder Harnstoff- und/oder Biuret- und/oder Isocyanurat- sowie besonders auch Ethergruppen enthaltenden NCO-Verbindung mit direkt an aromatische Reste gebundenen NCO-Gruppen entsteht, $R^1$ und $R^2$ unabhängig voneinander einen 1 bis 10 Kohlenstoffatome enthaltenden, linearen oder verzweigten oder cyclischen gesättigten Kohlenwasserstoffrest bedeuten oder zusammen einen Ring mit 4 bis 6 Ring-C-Atomen bilden und n eine ganze Zahl von 2 bis 4 bedeutet, einer basischen Hydrolyse unterworfen wird.

2. Verfahren zur Herstellung von Urethan- und/oder Harnstoff- und/oder Biuret- und/oder Allophanat- und/oder Isocyanurat- sowie bevorzugt Ethergruppen enthaltenden aromatischen Polyaminen, dadurch gekennzeichet, dass eine Verbindung der allgemeinen Formel

$A\text{-}(NCO)_n$,

wobei

A ein Rest ist, wie er durch Entfernung von n Isocyanatgruppen aus einer Urethan- und/oder Harnstoff- und/oder Biuret- und/oder Allophanat- und/oder Isocyanurat- und Ethergruppen enthaltenden, aromatisch gebundene NCO-Gruppen aufweisenden Verbindungen entsteht, und

n eine ganze Zahl von 2 bis 4 ist, gegebenenfalls gelöst in einem NCO-inerten Lösungsmittel, in der Wärme mit einem Gemisch aus

a) Wasser

b) 0,1 bis 10 Äquivalenten (bezogen auf 1 Äquivalent NCO) eines sekundären Amins der allgemeinen Formel

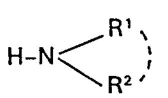

wobei $R^1$ und $R^2$ unabhängig voneinander jeweils einen 1 bis 10 Kohlenstoffatome enthaltenden linearen oder verzweigten aliphatischen oder cycloaliphatischen, gesättigten Kohlenwasserstoffrest bedeuten oder zusammen für einen 4 bis 6 Ring-C-Atome enthaltenden heterocyclischen Ring stehen,

c) 0 bis 2 Äquivalenten einer $OH^{\ominus}$-Gruppen besitzenden Base (bezogen auf 1 Äquivalent NCO),

d) 0 bis 2 Äquivalenten einer t-Aminogruppen aufweisenden Verbindung (bezogen auf 1 Äquivalent NCO) und

e) 0 bis 100 Teilen eines Lösungsmittels (bezogen auf 1 Teil der Verbindung $A\text{-}(NCO)_n$, worin A und n wie oben definiert sind),

wobei von den Komponenten c) und d) mindestens eine in einer Menge von 0,1 bis 2 Äquivalenten zum Einsatz kommt, behandelt und das entstandene aromatische Polyamin auf an sich bekannte Weise aus dem Reaktionsprodukt isoliert wird.

3. Verfahren nach Anspruch 1, dadurch gekennzeichnet, dass die basische Hydrolyse mit wässrigen Alkalihydroxiden vorgenommen wird.

4. Verfahren nach Anspruch 1, dadurch gekennzeichnet, dass $R^1$ und $R^2$ unabhängig voneinander Methyl, Ethyl, n-Propyl, i-Propyl, n-Butyl, i-Butyl und sec.-Butyl bedeuten.

5. Verfahren nach Anspruch 1, dadurch gekennzeichnet, dass als Verbindung der allgemeinen Formel

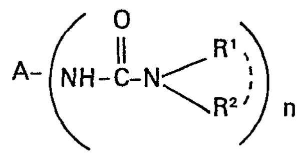

worin

A, $R^1$, $R^2$ und n wie oben definiert sind, ein Reaktionsprodukt aus einem sekundären Amin der allgemeinen Formel

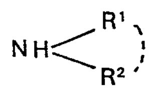

worin

$R^1$ und $R^2$ wie oben definiert, und einer aromatisch gebundene NCO-Gruppen aufweisenden Verbindung der allgemeinen Formel

$A\text{-}(NCO)_n$

worin

A und n wie oben definiert sind, eingesetzt wird.

6. Verfahren nach Ansprüchen 1 bis 5, dadurch gekennzeichnet, dass als NCO-Gruppen aufweisende Verbindung ein Umsetzungsprodukt aus einem 2 bis 4 Hydroxylgruppen aufweisenden Polyether mit einem Molekulargewicht von 400 bis 6000 sowie gegebenenfalls einem Polyol mit einem Molekulargewicht zwischen 62 und 399 und einem aromatischen Polyisocyanat im NCO:OH-Verhältnis von 1,5:1 bis 2,8:1 eingesetzt wird.

7. Verfahren nach Ansprüchen 1 bis 6, dadurch gekennzeichnet, dass als Komponente c) 1,0 bis 1,5 Äquivalente eines Alkalimetallhydroxids (bezogen auf 1 Äquivalent NCO) verwendet werden.

8. Verfahren nach Ansprüchen 1 bis 6, dadurch gekennzeichnet, dass in Komponente d) 1,0 bis 1,5 Äquivalente tert.-Amingruppen auf 1 Äquivalent NCO kommen.

9. Verfahren zur Herstellung von Urethan- und Ethergruppen aufweisenden Verbindungen, dadurch gekennzeichnet, dass eine aus Dimethylamin oder Diethylamin und einem NCO-Prepolymer, das erhalten wurde durch Umsetzung von einem zwei oder drei Hydroxylgruppen aufweisenden Polyether mit einem Molekulargewicht von 400 bis 6000 sowie gegebenenfalls einem Polyol mit einem Molekulargewicht zwischen 62 und 400 und einem aromatischen Polyisocyanat im NCO:OH-Verhältnis von 1,5:1 bis 2,8:1, hergestellte Harnstoffverbindung in Wasser mit einer der Menge der Harnstoffgruppen mindestens äquivalenten oder leicht überschüssigen Menge eines Alkalihydroxids bei erhöhter Temperatur behandelt und das aminische Endprodukt auf an sich bekannte Weise aus dem Reaktionsgemisch entfernt wird.

10. Verwendung der nach Verfahren 1 bis 9 hergestellten Urethan- und/oder Harnstoff- und/oder Biuret- und/oder Allophanat- und/oder Isocyanurat- sowie besonders auch Ethergruppen enthaltenden aromatischen Amine zur Herstellung von gegebenenfalls zellförmigen Polyurethankunststoffen und Polyurethanschaumstoffen durch Umsetzung von

I Polyisocyanaten mit
II Polyaminen sowie gegebenenfalls
III weiteren niedermolekularen und/oder höhermolekularen Verbindungen mit gegenüber Isocyanaten reaktiven Gruppen, gegebenenfalls
IV in Anwesenheit an sich bekannter Hilfs- und Zusatzstoffe,

dadurch gekennzeichnet, dass als Komponente II die gemäss Verfahren 1 bis 9 hergestellten Polyamine eingesetzt werden.

**Claims**

1. Process for the preparation of aromatic polyamines containing urethane and/or urea and/or allophanate and/or biuret and/or isocyanurate and preferably also ether groups, characterised in that a compound of the general formula

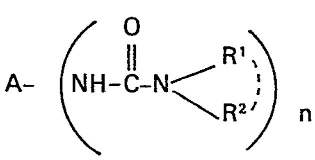

wherein
A is a radical such as is formed by removing n isocyanate groups from an NCO compound which contains urethane and/or urea and/or biuret and/or isocyanurate and particularly also ether groups and which has NCO groups directly bonded to aromatic radicals,
$R^1$ and $R^2$, independently of one another, denote a linear or branched or cyclic saturated hydrocarbon radical which contains 1 to 10 carbon atoms or together form a ring with 4 to 6 C atoms and
n denotes an integer from 2 to 4, is subjected to basic hydrolysis.

2. Process for the preparation of aromatic polyamines containing urethane and/or urea and/or biuret and/or allophanate and/or isocyanurate and preferably ether groups, characterised in that a compound of the general formula

$A\text{-}(NCO)_n$,

wherein
A is a radical such as is formed by removing n isocyanate groups from a compound containing urethane and/or urea and/or biuret and/or allophanate and/or isocyanurate and ether groups and having aromatically bonded NCO groups, and
n is an integer from 2 to 4, optionally dissolved in an NCO-inert solvent, is treated, while heating, with a mixture of

a) water,
b) 0.1 to 10 equivalents (based on 1 equivalent of NCO) of a secondary amine of the general formula

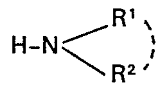

wherein
$R^1$ and $R^2$, independently of one another, each denote a linear or branched aliphatic or cycloaliphatic, saturated hydrocarbon radical which contains 1 to 10 carbon atoms or together represent a heterocyclic ring containing 4 to 6 ring C atoms,
c) 0 to 2 equivalents of a base containing $OH^{\ominus}$ groups (based on 1 equivalent of NCO),
d) 0 to 2 equivalents of a compound containing t-amino groups (based on 1 equivalent of NCO) and
e) 0 to 100 parts of a solvent (based on 1 part of the compound $A\text{-}(NCO)_n$, wherein A and n are as defined above),

at least one of components c) and d) being used in a quantity of 0.1 to 2 equivalents, and the aromatic polyamine formed is isolated from the reaction product in a manner known per se.

3. Process according to Claim 1, characterised in that the basic hydrolysis is carried out with aqueous alkali metal hydroxides.

4. Process according to Claim 1, characterised in that $R^1$ and $R^2$, independently of one another, denote methyl, ethyl, n-propyl, i-propyl, n-butyl, i-butyl and sec.-butyl.

5. Process according to Claim 1, characterised in that, as the compound of the general formula

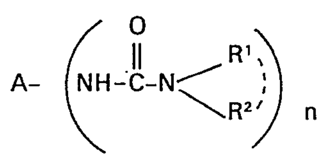

wherein
A, $R^1$, $R^2$ and n are as defined above, a reaction product of a secondary amine of the general formula

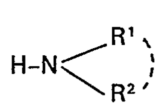

wherein
$R^1$ and $R^2$ as defined above, and a compound, containing aromatically bonded NCO groups, of the general formula

$A\text{-}(NCO)_n$

wherein
A and n are as defined above, is used.

6. Process according to Claims 1 to 5, characterised in that a reaction product of a polyether containing 2 to 4 hydroxyl groups and having a

molecular weight of 400 to 6,000 and optionally a polyol having a molecular weight between 62 and 399 and an aromatic polyisocyanate, in an NCO:OH ratio of 1.5:1 to 2.8:1, is used as the compound containing NCO groups.

7. Process according to Claims 1 to 6, characterised in that 1.0 to 1.5 equivalents of an alkali metal hydroxide (based on 1 equivalent of NCO) is used as component c).

8. Process according to Claims 1 to 6, characterised in that in component d) there are 1.0 to 1.5 equivalents of tert.-amino groups to 1 equivalent of NCO.

9. Process for the preparation of compounds containing urethane and ether groups, characterised in that a urea compound prepared from dimethylamine or diethylamine and an NCO prepolymer which has been obtained by reacting a polyether containing two or three hydroxyl groups and having a molecular weight of 400 to 6,000 and optionally a polyol having a molecular weight between 62 and 400 and an aromatic polyisocyanate, in an NCO:OH ratio of 1.5:1 to 2.8:1, is treated in water with a quantity of an alkali metal hydroxide which is at least equivalent to or slightly in excess of the quantity of urea groups, at an elevated temperature, and the aminic end product is removed from the reaction mixture in a manner known per se.

10. Use of the aromatic amines prepared by processes 1 to 9 and containing urethane and/or urea and/or biuret and/or allophanate and/or isocyanurate and particularly also ether groups for the preparation of optionally cellular polyurethane plastics and polyurethane foams by the reaction of

I polyisocyanates with
II polyamines and optionally
III other low molecular weight and/or higher molecular weight compounds containing groups reactive towards isocyanates, optionally
IV in the presence of auxiliaries and additives known per se,

characterised in that the polyamines prepared according to processes 1 to 9 are used as component II.

**Revendications**

1. Procédé de préparation de polyamines aromatiques contenant des groupes uréthane et/ou urée et/ou allophanate et/ou biuret et/ou isocyanurate, ainsi que, de préférence, également des groupes éther, caractérisé en ce qu'on soumet, à une hydrolyse basique, un composé de formule générale:

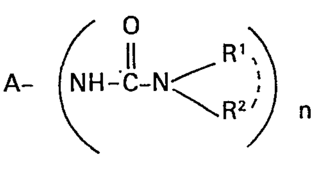

dans laquelle
A représente un radical du type obtenu par élimination de n groupes isocyanate d'un composé de NCO comportant des groupes NCO liés directement à des radicaux aromatiques, ce composé contenant des groupes uréthane et/ou urée et/ou biuret et/ou isocyanurate, ainsi que, en particulier, également des groupes éther,
$R^1$ et $R^2$ représentent chacun indépendamment l'un de l'autre un radical d'hydrocarbure saturé linéaire, ramifié ou cyclique contenant 1 à 10 atomes de carbone ou, ensemble, ils forment un noyau contenant 4 à 6 atomes de carbone cycliques, et
n représente un nombre entier de 2 à 4.

2. Procédé de préparation de polyamines aromatiques contenant des groupes uréthane et/ou urée et/ou biuret et/ou allophanate et/ou isocyanurate, ainsi que, de préférence, des groupes éther, caractérisé en ce qu'on traite un composé de formule générale:

$$A\text{-}(NCO)_n$$

dans laquelle
A représente un radical du type obtenu par élimination de n groupes isocyanate d'un composé comportant des groupes NCO à liaison aromatique et contenant des groupes uréthane et/ou urée et/ou biuret et/ou allophanate et/ou isocyanurate, ainsi que des groupes éther, et
n est un nombre entier de 2 à 4, éventuellement en solution dans un solvant inerte vis-à-vis des groupes NCO, à chaud, avec un mélange comprenant:

a) de l'eau
b) 0,1 à 10 équivalents (calculé sur 1 équivalent de NCO) d'une amine secondaire de formule générale:

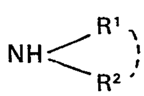

dans laquelle $R^1$ et $R^2$ représentent chacun indépendamment l'un de l'autre un radical d'hydrocarbure saturé linéaire ou ramifié, aliphatique ou cycloaliphatique contenant 1 à 10 atomes de carbone ou, ensemble, ils représentent un noyau hétérocyclique contenant 4 à 6 atomes de carbone cycliques,
c) 0 à 2 équivalents d'une base comportant des groupes $OH^{\ominus}$ (calculé sur 1 équivalent de NCO),
d) 0 à 2 équivalents d'un composé comportant des groupes t-amino (calculé sur 1 équivalent de NCO) et
e) 0 à 100 parties d'un solvant (calculé sur 1 partie du composé $A\text{-}(NCO)_n$ où A et n ont les significations définies ci-dessus),

en utilisant au moins un des composants c) et d) en une quantité de 0,1 à 2 équivalents, et on isole la polyamine aromatique formée du produit réactionnel de façon connue en soi.

3. Procédé selon la revendication 1, caractérisé en ce qu'on effectue l'hydrolyse basique avec des hydroxydes alcalins aqueux.

4. Procédé selon la revendication 1, caractérisé en ce que $R^1$ et $R^2$ représentent chacun indépendamment l'un de l'autre un groupe méthyle, un groupe éthyle, un groupe n-propyle, un groupe i-propyle, un groupe n-butyle, un groupe i-butyle et un groupe sec-butyle.

5. Procédé selon la revendication 1, caractérisé en ce que, comme composé de formule générale:

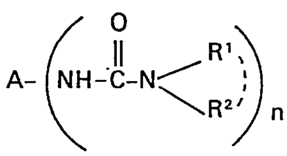

dans laquelle
A, $R^1$, $R^2$ et n ont les significations définies ci-dessus, on utilise un produit réactionnel d'une amine secondaire de formule générale:

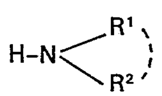

dans laquelle
$R^1$ et $R^2$ ont les significations définies ci-dessus, et d'un composé comportant des groupes NCO à liaison aromatique et répondant à la formule générale:

$A{-}(NCO)_n$

dans laquelle
A et n ont les significations définies ci-dessus.

6. Procédé selon les revendications 1 à 5, caractérisé en ce que, comme composé comportant des groupes NCO, on utilise un produit réactionnel d'un polyéther comportant 2 à 4 groupes hydroxy et ayant un poids moléculaire de 400 à 6000, ainsi qu'éventuellement d'un polyol ayant un poids moléculaire se situant entre 62 et 399 et d'un polyisocyanate aromatique dans le rapport NCO:OH de 1,5:1 à 2,8:1.

7. Procédé selon les revendications 1 à 6, caractérisé en ce que, comme composant c), on utilise 1 à 1,5 équivalent d'un hydroxyde d'un métal alcalin (calculé sur 1 équivalent de NCO).

8. Procédé selon les revendications 1 à 6, caractérisé en ce que, dans le composant d), on a 1 à 1,5 équivalent de groupes tert.-amino pour 1 équivalent de NCO.

9. Procédé de préparation de composés comportant des groupes uréthane et éther, caractérisé en ce qu'on traite un composé d'urée préparé à partir de diméthylamine ou de diéthylamine et d'un prépolymère de NCO que l'on a obtenu par réaction d'un polyéther comportant deux ou trois groupes hydroxy et ayant un poids moléculaire de 400 à 6000, ainsi qu'éventuellement d'un polyol ayant un poids moléculaire se situant entre 62 et 400 et d'un polyisocyanate aromatique dans le rapport NCO:OH de 1,5:1 à 2,8:1, dans l'eau avec un hydroxyde alcalin en une quantité légèrement excédentaire ou équivalent au moins à la quantité des groupes urée, à température élevée, puis on élimine le produit final aminique du mélange réactionnel de façon connue en soi.

10. Utilisation des amines aromatiques préparées selon les procédés des revendications 1 à 9 et contenant des groupes uréthane et/ou urée et/ou biuret et/ou allophanate et/ou isocyanurate, ainsi que, en particulier, également des groupes éther pour la fabrication de mousses de polyuréthanes et de matières synthétiques de polyuréthanes éventuellement cellulaires par réaction:

I. de polyisocyanates avec
II. des polyamines, ainsi qu'éventuellement
III. d'autres composés de faible poids moléculaire et/ou de poids moléculaire très élevé comportant des groupes réactifs vis-à-vis des isocyanates, éventuellement
IV. en présence de substances auxiliaires et d'additifs connus en soi,

caractérisées en ce que, comme composant II, on utilise les polyamines préparées selon les procédés des revendications 1 à 9.